# EUROPEAN PATENT SPECIFICATION

(11) **EP 1 816 955 B1**
(45) Date of publication and mention of the grant of the patent: **05.04.2017**
(21) Application number: 05852771.4
(22) Date of filing: 02.12.2005
(51) Int. Cl.: A61B 5/04, A61B 5/00, G06F 19/00

(54) **REMOTE ELECTROCARDIOGRAPHIC MONITORING**
FERNÜBERWACHUNG DES EKG
SURVEILLANCE À DISTANCE D'ÉLECTROCARDIOGRAMME

(30) Priority: 02.12.2004 US 4311
(43) Date of publication of application: 15.08.2007
(73) Proprietor: CardioNet, Inc., Malvern, PA 19355 (US)
(72) Inventor: EVELAND, Doug C., Bonsall, California 92003 (US)
(74) Representative: Pisani, Diana Jean
(86) International application number: PCT/US2005/043652
(87) International publication number: WO 2006/060669

(56) References cited:
- WO-A1-02/067122
- US-A- 5 339 821
- US-A1- 2002 019 586
- US-A1- 2002 198 473
- US-A1- 2003 036 683
- CORDISCO M E ET AL: "Use of telemonitoring to decrease the rate of hospitalization in patients with severe congestive heart failure.", THE AMERICAN JOURNAL OF CARDIOLOGY, vol. 84, no. 7, 1 October 1999 (1999-10-01), pages 860-862 , A8, XP002626288, ISSN: 0002-9149
- GÓMEZ E J ET AL: "Telemedicine as a tool for intensive management of diabetes: the DIABTel experience.", COMPUTER METHODS AND PROGRAMS IN BIOMEDICINE, vol. 69, no. 2, August 2002 (2002-08), pages 163-177, XP002626289, ISSN: 0169-2607

## Description

### BACKGROUND

The following description relates to controlling access to a medical monitoring device and/or a service associated with the device, for example, to help ensure that access to the monitoring device and service is authorized prior to commencing usage.

Advances in sensor technology, electronics, and communications have made it possible for physiological characteristics of patients to be monitored even when the patients are ambulatory and not in continuous, direct contact with a hospital monitoring system. For example, U.S. Patent 5,959,529 describes a monitoring system in which the patient carries a remote monitoring unit with associated physiological sensors. The remote monitoring unit conducts a continuous monitoring of one or more physiological characteristics of the patient according to the medical problem of the patient, such as the patient's heartbeat and its waveform.

One potential issue associated with the use of such medical monitoring devices is establishing whether the patient's health-care-benefit payer has authorized the use of the monitoring device and service. In the absence of a proper authorization, the patient may use the medical monitoring device and incur significant charges, for example, in the form of rental value of the medical monitoring device, telephone charges, charges at the central monitoring system, and charges by medical personnel, and the providers of those goods and services may not get paid. Bad debts - an increasing concern in the medical field generally - tend to be an even greater concern in the case of a portable medical monitoring device and its service where the physical control of the device is in the hands of a third party, such as a prescribing doctor, who does not own the medical monitoring device and is not responsible for improper charges.

U.S. 2003/036683 A1 discloses an arrangement in which diagnostic modules can analyze data collected in response to a patient query transmitted to a medical monitoring device. If patient monitoring reveals a glucose level that is dangerously high, the medical monitoring device or a central monitoring device can determine the need for cardiovascular monitoring and initiate blood pressure and cardiac status monitoring. WO 02/067122 A1 discusses monitoring patient variables in a wireless manner via a patient worn monitoring device. U.S. 5,339,821 discusses a home medical system including equipment for measuring the electrocardiogram and other heart conditions of a user, a display for explaining the procedure and a result of a measurement, and a communication link connecting the user's equipment to a medical institution.

### SUMMARY

In an aspect, a method performed by a medical monitoring device system comprises monitoring a medical condition of an individual, the monitoring being initiated remotely by a monitoring service, wherein monitoring the medical condition comprises electrocardiographic monitoring of the individual, receiving, from the monitoring service at the medical monitoring device system, a query relating to a diabetic condition of the individual, transmitting a response to the query from the medical monitoring device system to the monitoring service, receiving a prompt from the monitoring service, the prompt designed to provoke a predetermined action, and adapting the monitoring of the medical condition in accordance with the received parameters, the adapted parameters being based on the received response to the query regarding the diabetic condition of the individual..

This and other aspects can include one or more of the following features. A response to the prompt can be transmitted to the monitoring service. The received prompt can be designed to provoke the monitored individual to undertake the predetermined action.

Receiving the prompt can include receiving, for example, a medication prompt designed to provoke the monitored individual to medicate, a sample prompt designed to provoke the collection of a sample, an electrode check prompt designed to move the monitored individual to check an electrode, or a communications check prompt designed to move the monitored individual to ensure that communications with the monitoring service are maintained. The prompt can also be a battery check prompt designed to move the monitored individual to check a charge on the battery, a physical activity prompt designed to move the monitored individual to curtail or halt physical activity, or a prompt designed to move the monitored individual to lie down or sit down.

The aspect can also include receiving input from the monitored individual and using the input in responding to the query.

In another aspect, there is provided an article including a machine-readable medium storing instructions operable to cause one or more devices to perform operations comprising: monitoring a first medical condition of an individual, where monitoring the medical condition comprises electrocardiographic monitoring of the individual, receiving a query from the monitoring service, the query relating to a diabetic condition of the individual, transmitting a response to the query to the monitoring service, transmitting a result of the monitoring to the monitoring service, receiving an activity prompt from the monitoring service, the activity prompt relating to an activity that is to be prompted; prompting the activity related to the activity prompt, transmitting a response to the prompt to the monitoring service, receiving, from the monitoring service, adapted parameters for the monitoring of the medical condition of the individual, the adapted parameters being based on the response to query relating to the diabetic condition of the individual, and adapting the monitoring of the first medical condition in accordance with the received parameters.

This and other aspects can include one or more of the following features. The activity can be prompted by relaying the prompt to the monitored individual and receiving a response to the relayed prompt from the monitored individual. Prompting activity may comprise provoking the collection of a sample. For example, the collection of the result of the monitoring can be provoked. The monitored individual can be provoked to medicate with a specific medication. For example, the monitored individual can be provoked to medicate using a pharmaceutical composition having a specific color.

The response can be transmitted without input from the monitored individual.

In another aspect, a method comprises: receiving a monitoring result from a remote monitoring device, the remote monitoring device remotely monitoring a medical condition of an individual, wherein the remote monitoring device is adapted for electrocardiographic monitoring of the individual; receiving, from the remote monitoring device, a response to a query regarding a diabetic condition of the individual; and adapting the monitoring of the medical condition of the individual by the remote monitoring device with adapted parameters, the adapted parameters being based on the received response to the query regarding the diabetic condition of the individual.

This and other aspects can include one or more of the following features. Receiving the response can include receiving a response to an individual-specific query tailored to the monitored individual. The monitoring result and the response can be stored. To adapt the monitoring, adapted monitoring parameters can be transmitted to the remote monitoring device. For example, adapted parameters for the identification of cardiac states can be transmitted to the remote monitoring device. The parameters can be adapted to change thresholds at which a first disease state is identified. The parameters can be adapted to change the algorithms used in monitoring. The aspect can further include distributing the response to one or more third parties to whom the response is relevant in response to receipt of the response.

In another aspect, a device includes an electrocardiograph configured to remotely monitor cardiac function of an individual; a receiver configured to receive a communication from a remote monitoring service, wherein the receiver is configured to receive a query relating to a diabetic medical condition of the individual; an output device configured to relay the communication to the monitored individual, where the output device is configured to display the query; an input device configured to receive an input responsive to the relayed communication from the monitored individual; and a transmitter configured to transmit a response to the communication to the remote monitoring service, the response based at least in part on the input received from the monitored individual, wherein the receiver is configured to receive, from the monitoring service, adapted parameters for adapting the monitoring of the cardiac function of the individual based on the response to the query relating to the diabetic condition of the individual

This aspect can include one or more of the following features. The receiver can be configured to receive a prompt designed to move the monitored individual to a particular action.

The details of one or more implementations are set forth in the accompanying drawings and the description below. Other features will be apparent from the description and drawings, and from the claims.

### DESCRIPTION OF DRAWINGS

FIG. 1 is a flow diagram of a method of controlling access to a medical monitoring device and/or an associated service.
FIGS. 2A and 2B are schematic illustrations of medical monitoring systems for implementing the method of FIG. 1.
FIG. 3 is a flowchart of a process that can be performed by a medical monitoring device system during its term of service in an embodiment.
FIG. 4 shows a table of example medical queries that can be handled during the term of service of a medical monitoring device.
FIG. 5 is a flowchart of a process that can be performed by a medical monitoring device system during its term of service included in an embodiment.
FIG. 6 shows a table of example activity prompts that can be handled during the term of service of a medical monitoring device.
FIG. 7 shows a data store for storing information during the term of service of a medical monitoring device.
FIG. 8 is a flowchart of a process that can be performed by a central unit in response to receipt of information from a medical monitoring device system included in an embodiment.
FIG. 9 is a schematic illustration of another implementation of a medical monitoring system in which an embodiment may be implemented.
FIG. 10 is a flowchart of a process that can be performed by a electrocardiograph monitoring system during a clinical trial outside the scope of the present claims.

Like reference symbols in the various drawings indicate like elements.

### DETAILED DESCRIPTION

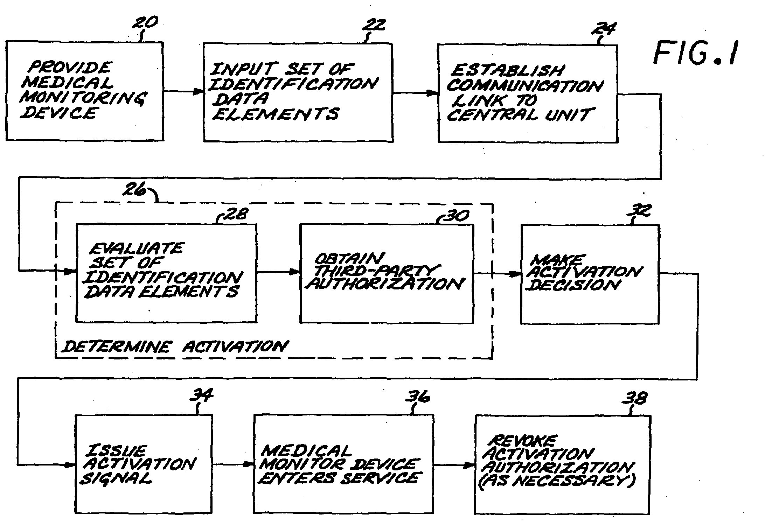

FIG. 1 depicts a flow diagram of a method of controlling access to a medical monitoring device and/or service. A medical monitoring device and its associated system are provided (20). The medical monitoring device and medical monitoring system may be of any operable type, such as that disclosed in US Patent 5,959,529 (hereafter, "the `529 patent").

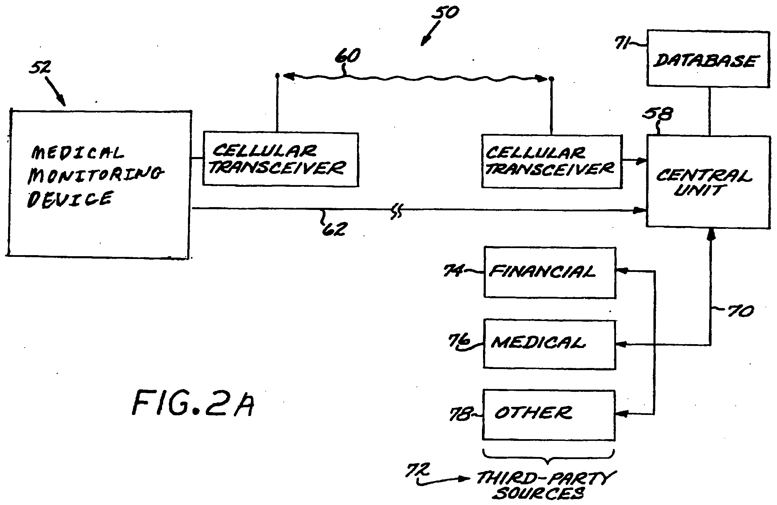

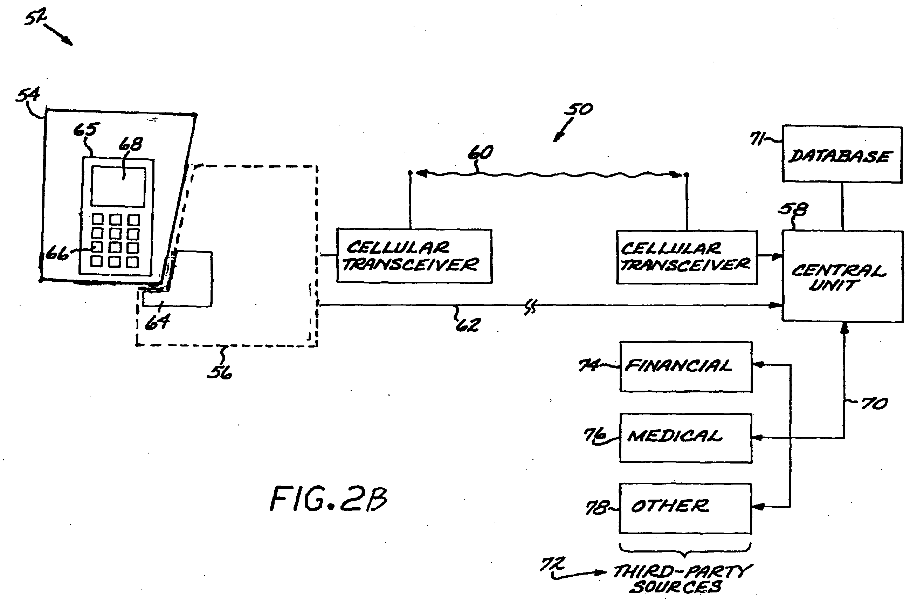

FIGS. 2A and 2B depict details, in block diagram form, of medical monitoring systems 50 that include a medical monitoring device system 52. Medical monitoring device systems 52 include one or more medical monitoring devices that monitor a biological parameter. A biological parameter is a quantity that characterizes an aspect of a biological system. In the medical context, biological parameters generally include information relating to the physiological state of an organism. Examples of medical monitoring devices include devices that measure electrical potentials (e.g., electrocardiography (ECG's), electromyography, and electroencephalography devices), devices that measure blood and other body fluid analyte constituents (e.g., pulse oximetry devices and devices that measure blood glucose concentration, blood pH, and/or other ion concentrations), and devices that measure mechanical characteristics (e.g., blood pressure transducers, heart sound transducers, height and/or weight measurement devices).

In FIG. 2B, medical monitoring device system 52 includes both a medical monitoring device 54 and a base station 56. The medical monitoring device 54 may be a portable or remote monitoring unit of the type generally described in the '529 patent. The base station 56 has communication access to a central unit 58 through a communication link such as a wireless cellular telephone transceiver link 60 and/or a telephone land-line 62. Alternatively, or in addition, communication links can be established by other available means, among others, such as wired or wireless networks that implement communications protocols and standards such IP (Internet protocol), WiFi (IEEE 802.11x), WiMax (IEEE 802.16x), and GPRS (General Packet Radio Service). The central unit 58, typically maintained at a central node or location, may in practice be composed of multiple computer systems distributed across, or even outside of, the central node.

In other implementations, medical monitoring device 54 can have direct communication access to central unit 58 through a communication link. For example, medical monitoring device 54 can include a cellular transceiver to establish a direct wireless cellular telephone transceiver link to central unit 58.

In the implementation shown in FIG 2B, the base station 56 has a base station cradle 64 that is configured to receive the medical monitoring device 54. The medical monitoring device 54 includes an input/output device 65 that typically has a microprocessor, communications controller, and communications hardware and/or software to establish the links 60 and/or 62. The input/output device 65 has a keypad 66 for inputting information and a display 68 to view the input information and other information to be displayed, as well as information transmitted to the input/output device 65. Alternatively, input/output device 65 can use a touch-sensitive display without dedicated keys to interact with a user.

In the implementations shown in FIGS. 2A and 2B, the central unit 58 has communications access to a variety of databases 71 and to third-party sources 72, typically by telephone land-line, network or other connection 70. The databases 71 may include prior patient records, general records, and the like. The third-party sources 72 may include, for example, financial sources 74, medical sources 76, and other sources 78. A financial source might be, for example, an insurance company, the social-security administration, or a credit-granting company. A medical source might be, for example, a specialist physician whose authorization is required before commencing the monitoring of the patient. Other third-party sources might be, for example, the company that maintains the medical monitoring device and which is consulted to be certain that the specific medical monitoring device to be activated is approved for service.

In the implementation shown in FIG 2B, the base station 56, when not prescribed or otherwise assigned and distributed to a patient to be monitored, ordinarily is in the custody the office of the agency that is providing the medical monitoring device 54 to a patient for the purpose of monitoring physiological parameters of the patient. Such an agency could be, for example, the patient's physician or a hospital. When the agency undertakes to provide the medical monitoring device 54 to the patient, for example, to take home, the medical monitoring device 54 is docked with the base station 56, and the procedures described in relation to subsequent portions of FIG 1 are followed.

Returning to FIG. 1, a set of identification data elements (22) are input into the medical monitoring device system 52. For example, a set of identification data elements can be input through the keypad 66 of the input/output device 65 of the medical monitoring device 54 of FIG. 2B. The identification data elements may include, for example, one or more of a patient name, a patient address, a patient social security number, a patient sex, and an identification of the third-party financial source. In one implementation, a set-up sequence may involve the input of a patient number or other patient identifier. The input patient identifier can be used to retrieve other information about the patient, including name, address, social security number, sex, and the identity of a third-party financial source. The identifier of the medical monitoring device 54, such as its serial number, may be manually input in step 22, but more typically the identifier is automatically made available by the medical monitoring device 54 to the base station 56.

The input/output device 65 may perform a preliminary evaluation of the set of identification data elements, for example, such as to determine by using a software utility program whether the identification data elements meet a set of one or more format requirements. Such basic format requirements may be specified for each of the identification data elements. For example, a format requirement may specify that a patient name is to include only alphanumeric characters. If as typed into the keyboard the patient name includes other characters (e. g., a percent sign %), software running in the input/output device 65 can recognize the error and provide an input diagnostic message through the display 68 to prompt the input of correct information. In another example, a format requirement may specify that a user's social security number must contain 10 numerical digits and may not contain letters or other characters.

After what appears from the preliminary format evaluation to be a set of correct identification data elements is input to the input/output device 65, the medical monitoring device system 52 establishes (24) a communication link to the central unit 58. The communication link is preferably through the land-line 62, but may be though the cellular telephone transmission link 60 or another wireless or wired link if the land-line is not available.

The medical monitoring device system 52 and the central unit 58 cooperatively determine whether the medical monitoring device may be activated for rendering medical monitoring device service (26). The final decision is typically made by the central unit 58, although the medical monitoring device system 52 may aid in data processing or may be called upon for additional input, for example, such as when the patient name is found not to match with the social security number in other records.

The activation determination process 26 may include various sub-processes including evaluating the set of identification data elements as to whether they meet a set of basic structural requirements (28) and obtaining third-party authorization (30) from one or more of the third-party sources 72. The sub-processes of evaluating 28 and obtaining 30 are preferably performed automatically. "Automatically" as used herein indicates that the steps are performed without human action or intervention, except where a discrepancy occurs. The present system is organized to perform the evaluating and obtaining steps entirely by computer procedures, to minimize costs and take advantage of data collections at a variety of locations. Alternatively, the present approach may be performed in whole or in part using manual (i.e., human-performed) sub-processes 28 and 30. In addition, the sub-process 30 of obtaining third-party authorization can be performed either before or during the activation determination process 26. For example, the sub-process 30 may involve updating a locally stored (e.g., at a central unit hosting the medical monitoring service) copy of a third party's database of authorization information, and then performing the sub-process 30 by accessing the local copy of the database instead of accessing the third party's system, which typically would be maintained at a remote location. Updating of the local third-party authorization database may occur based on one or both of the following criteria:
(i) periodically (e.g., once a day) or (ii) based on an occurrence of a predetermined event (e.g., the third-party system determines that a certain amount of new authorization data is available and has not yet been copied to the central unit's local database and/or the central unit determines that its local database does not contain needed information).

The set of basic structural requirements to be imposed may include the format requirements evaluated by the input/output device 65, or may include different or additional structural requirements. For example, the central unit 58 may check the database 71 to attempt to match the input patient name with a social security number that is already in the database 71 from prior medical contacts. If the patient name and the social security number that were input in sub-process 22 do not match, then further inquiry may be made back to the medical monitoring device system 52. The failure to match the name and the social security number may arise from a simple inputting error, which can be corrected with revised input, or it may arise from a fraudulent attempt to obtain medical monitoring services that is detected by the procedures of sub-process 28.

As noted above, obtaining third-party authorization in sub-process 30 may include contacting appropriate third-party sources 72, e.g., either on a dynamic, as-needed basis, and/or ahead of time by periodically replicating a third party's remote database to create a local copy. The financial source 74 may be contacted to determine whether it authorizes the charges associated with the patient monitoring services. This authorization is particularly important for the business interests of the provider of the services, for example, to avoid unpaid billings. Unpaid billings for medical services represents a major loss for many medical service companies. The medical source 76 may be contacted to determine whether it authorizes the patient monitoring. For example, if the prospective patient is being treated by more than one physician, it may be important to obtain authorization from each physician who is treating the patient before medical monitoring services are commenced. In this case, "authorization" signals formal recognition by the authorizing party that monitoring information will be available. Other sources 78 may also be contacted to determine whether they authorize the patient monitoring. For example, it may be desirable to ensure that the company responsible for maintaining a specific medical monitoring device authorizes its use. If a prior user had reported a problem and the specific medical monitoring device had been taken out of service for repair, but was mistakenly to be re-activated without being repaired, the company responsible for the maintenance could prevent its activation at this stage.

Thus, the procedures in sub-process 26 act as a "sign off" by a number of checks and third-parties to minimize the possibility that a medical monitoring device will be wrongly issued to a patient and activated. If the sign-offs are not completed, the medical monitoring device is not activated until the reason for the non-completion may be investigated. It is expected that in the great majority of cases, the activation determination process 26 will be completed without incident and so rapidly that the checking will be transparent to the patient and the issuer of the medical monitoring device.

Based on results of the activation determination process, the activation decision is made (32). The final decision is typically made at the central unit 58, although all or part of the final decision could be made at the base station and/or distributed or made by a system at another location. Typically, the decision is made at the central unit 58 because it has the access to the required information during the activation determination process 26, and because the central unit 58 tends to be more immune to tampering than the input/output device 65.

In the event that the identification data elements meet the set of basic structural requirements and third-party authorization is obtained, the central unit 58 issues an activation signal (34) to the medical monitoring device system 52 over the communication link 60 or 62. The medical monitoring device is activated and enters service (36).

The "activation signal" may be of any operable type. It may be a software "on" switch that enables the processing of data within a microprocessor in the medical monitoring device or a hardware "on" switch that turns on particular hardware functions such as the communications links built into the medical monitoring device. The activation signal may be complex, and may include identification of the patient and the specific medical monitoring device that is associated with that patient. This activation signal may then be transmitted with each subsequent communication between the medical monitoring device and the central unit 58 for identification purposes. In the event that the proper activation signal is not transmitted with each communication, it may be ignored. The activation may be revoked (38) at a later time if the authorization is withdrawn or for other reasons. Upon revocation 38, the signals transmitted by the medical monitoring device are not acted upon, and the patient and/or the issuing authority are notified and requested to return the medical monitoring device. As an alternative to revocation 38, the activation signal of step 34 may include a maximum time limit for which activation is authorized, so that a further authorization is required to extend the period of authorized use.

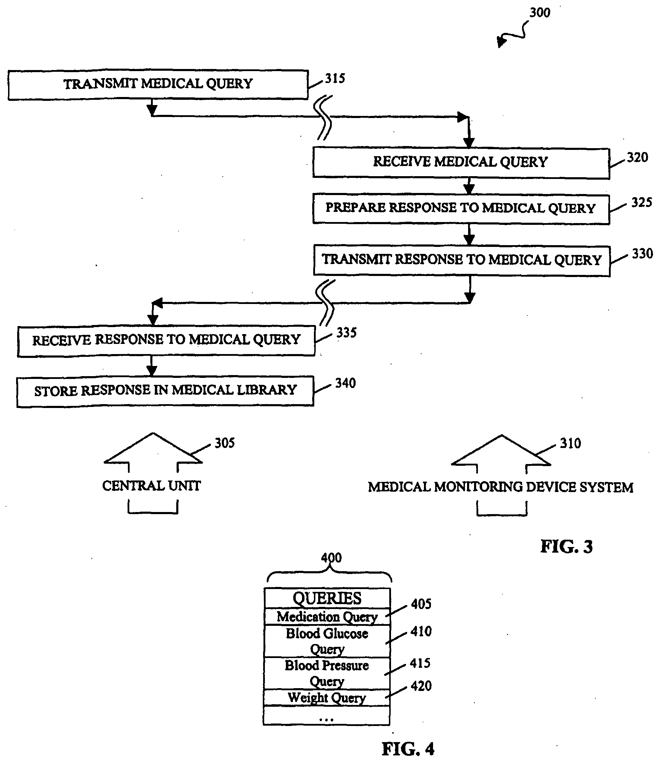

FIG. 3 shows a process 300 that can be performed during the term of service of a medical monitoring device. Aspects of process 300 performed by a central unit such as central unit 58 are denoted by arrow 305, whereas aspects of process 300 performed by a medical monitoring device such as device 54 are denoted by arrow 310. In process 300, a central unit sends a medical query to a medical monitoring device system at 315. A medical query is an inquiry regarding the medical condition of an individual who is monitored by a medical monitoring device.

FIG 4 shows a table 400 that identifies example medical queries 405, 410, 415, 420. Medical query 405 is an inquiry regarding the medication of an individual. For example, medical query 405 can inquire whether or not the individual has recently medicated with a particular medication or medical query 405 can inquire as to the dosage at which the individual has medicated. Medical query 410 is an inquiry regarding the individual's blood glucose concentration. Medical query 415 is an inquiry regarding the individual's blood pressure. Medical query 420 is an inquiry regarding the individual's weight.

Medical queries can be patient-specific in that they are relevant to the medical state of an individual or to classes of individuals. Medical queries can be tailored to the individual's medical condition, location, or activities. Medical queries can address a number of different disease states, including those outside the purview of the primary function of the medical monitoring device system. In accordance with the invention, the medical monitoring device system is an electrocardiograph, and medical queries include
queries directed to an individual's diabetic condition.

In addition, medical queries can be periodic queries made at certain fixed intervals during the monitoring of an individual. For example, periodic queries as to an individual's blood pressure can be made every four hours or so, whereas periodic queries as to an individual's weight can be made every three months or so. Medical queries can also be "one time" queries in that they are made during the onset of monitoring. Such queries can inquire as to the individual's age, gender, and medical history. Medical queries can also be "dynamic" queries in that the frequency at which the queries are made is adjusted based on the responses to previous queries or the responses to previous activity prompts, as discussed below. For example, when a response to a blood glucose query 410 indicates that the individual's blood glucose level is falling, the frequency of subsequent blood glucose queries 410 can be increased until the individual's blood glucose level ceases to fall or begins to increase.

Returning to FIG 3, the transmitted medical query is received at a medical monitoring device at 320. The medical query can be received over a wireless communication link and/or a wired communication link. The medical monitoring device can then prepare a response to the received medical query at 325. The preparation of the response can be performed automatically, i.e., without input from the individual who is monitored. For example, the response can be prepared using monitoring results read from a sensor. Alternatively, the response can be prepared based on input from the individual who is monitored. For example, the medical monitoring device system can present a text version of the medical query to the monitored individual. The medical monitoring device system can then receive information used to frame a response over one or more input devices.

Once the response is prepared, the medical monitoring device can transmit the prepared response to a central unit at 330. The response can be transmitted over a wireless communication link and/or a wired communication link. The central unit can receive the response to the medical query at 335. The received response can be stored in a medical library that includes other information relating to the medical condition of the monitored individual at 340. One implementation of such storage is discussed further below.

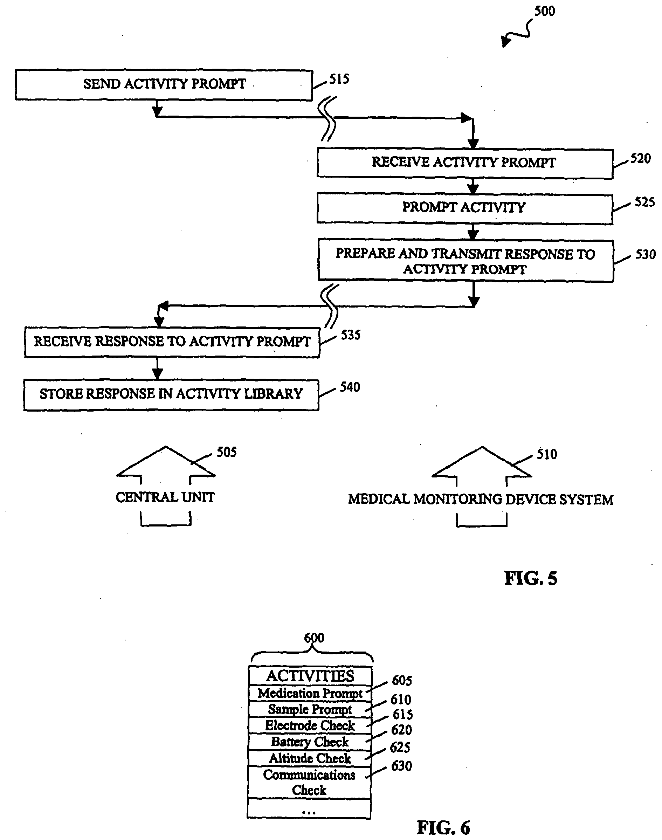

FIG. 5 shows a process 500 that can be performed during the term of service of a medical monitoring device system. Aspects of process 500 performed by a central unit such as central unit 58 are denoted by arrow 505, whereas aspects of process 500 performed by a medical monitoring device such as device 54 are denoted by arrow 510.

In process 500, a central unit sends an activity prompt to a medical monitoring device at 515. An activity prompt is a trigger designed to provoke a particular action. FIG. 6 shows a table 600 that identifies example activity prompts 605, 610, 615, 620, 625, 630. Activity prompt 605 is a trigger designed to provoke a monitored individual to medicate in a certain way. For example, activity prompt 605 can identify a medication and a dosage for the medication that is to be taken by the monitored individual. The medication can be identified, e.g., by name, by ID #, by shape, or by color. Medication can also be identified using a visual display device that presents an image of the medication (e.g., a purple, ovoid capsule) to identify the medication. Activity prompt 610 is a trigger designed to provoke the collection of a sample. For example, activity prompt 610 can provoke a monitored individual to take a urine sample or a blood sample. Activity prompt 610 can also prompt an individual to facilitate monitoring using a medical monitoring device system. For example, when the medical monitoring device system is a electrocardiograph, activity prompt 610 can prompt the monitored individual to ensure that electrodes are connected, to minimize the amount or type of movement, or to recline for collection of an electrocardiograph signal sample.

Activity prompt 615 is a trigger designed to provoke a monitored individual to check that any electrodes of the medical monitoring device are deployed properly. For example, activity prompt 615 can provoke a monitored individual to check that contact between the monitoring electrodes and the proper portion of and/or location on the body is maintained. Activity prompt 620 is a trigger designed to provoke a monitored individual to check that a battery or other power source for the medical monitoring device is sufficiently charged to properly supply the medical monitoring device with power. Activity prompt 625 is a trigger designed to provoke a monitored individual to check and decrease his or her altitude. In particular, individuals with certain medical conditions may be respond unfavorably to altitudes and activity prompt 625 may act to remind them to consider altitude during activities. Activity prompt 630 is a trigger designed to provoke a monitored individual to consider the likelihood of losing one or more communications links with the central unit. For example, activity prompt 630 can be triggered when a signal over a wireless communication link is fading.

Activity prompts can also be patient-specific in that they are relevant to the monitored individual or to classes of individuals. Activity prompts can be tailored to the individual's medical condition, location, or activities. Activity prompts can address a number of different disease states, including those outside the purview of the primary function of the medical monitoring device system. For example, when the medical monitoring device system is an electrocardiograph, activity prompts can include queries directed to an individual's participation in a clinical trial, as discussed below.

Activity prompts can also be periodic in that they are made a certain fixed intervals during the monitoring of an individual. For example, periodic prompts to medicate can be made in accordance with an individual's medication regimen. Activity prompts can also be "one time" prompts in that they are made during the onset of monitoring. Activity prompts can also be "dynamic" prompts in that the frequency at which the prompt are made is adjusted based on the responses to previous prompts or the responses to previous queries. For example, when responses to altitude check prompts 625 consistently indicate that an individual is at or near sea level, the frequency of altitude check prompts 410 can be decreased until an anomalous response is obtained.

Additional examples of activity prompts include triggers designed to provoke a monitored individual to, e.g., lie down, sit down, curtail or halt physical activity, commence or increase physical activity, apply pressure to a wound, contact a doctor, call an ambulance, decrease altitude, measure blood glucose level, measure blood pressure, take a diuretic, or hydrate.

Returning to FIG 5, the transmitted activity prompt is received at a medical monitoring device at 520. The activity prompt can be received over a wireless communication link and/or a wired communication link. The medical monitoring device can prompt the desired activity at 525. Such prompting can be directed to the monitored individual or to other elements in the medical monitoring device. In one implementation, a message is presented to the monitored individual over an output device such as an LCD screen. In another implementation, data collection elements in the medical monitoring device are directed to collect a certain sample.

The medical monitoring device can also prepare and transmit a response to the received activity prompt at 530. The preparation of the response can be done automatically, i.e., based on sensor readings and without input from the individual who is monitored. For example, when the medical monitoring device system is an electrocardiograph, the prepared response can include electrocardiogram data. Alternatively, the preparation of the response can be based on input from the individual who is monitored. For example, the medical monitoring device can present a text version of the activity prompt to the monitored individual and then receive a response over one or more input devices.

Once the response is prepared, the medical monitoring device system can transmit the prepared response to a central unit at 530. The response can be transmitted over a wireless communication link and/or a wired communication link. The central unit can receive the response to the activity prompt at 535. The received response can be stored in an activity library that includes other information relating to other activities of the monitored individual at 540.

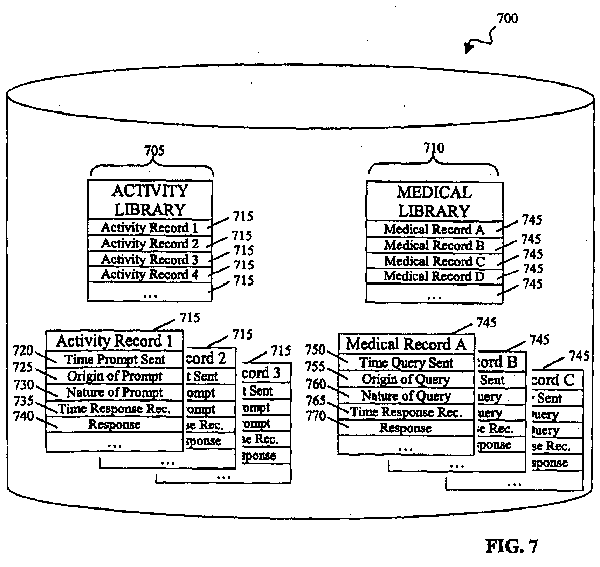

FIG 7 shows a data store 700 suitable for storing one or more activity libraries 705 and one or more medical libraries 710. Activity library 705 includes a collection of activity records 715 that describe individual activities that have been prompted by the central unit. For example, each activity record 715 can include a time prompt sent field 720, a prompt origin field 725, a nature of prompt field 730, a time response received field 735, and a response field 740. Time prompt sent field 720 includes information identifying the time when the prompt was transmitted from the central unit to the medical monitoring device system. Prompt origin field 725 includes information identifying the cause of the prompt. For example, an altitude check prompt may be sent when a monitored condition at the medical monitoring device system indicates that altitude may be a factor in the medical condition of the monitored individual. The monitored condition can be, e.g., a geographic location identified using a global positioning satellite (GPS) reading taken by the device.

Nature of prompt field 730 includes information identifying the subject of the transmitted prompt. For example, nature of prompt field 730 can identify the activity that was designed to be triggered. Time response received field 735 can include information identifying the time when a response to the prompt was received. Response field 740 can include information identifying the response received. For example, response field 740 can identify that the monitored individual has taken the prompted medication or that the monitored individual has collected a biological sample.

Medical library 710 includes a collection of medical records 745 that describe responses to queries by the central unit. For example, each medical record 745 can include a time query sent field 750, a query origin field 755, a nature of query field 760, a time response received field 765, and a response field 770. Time query sent field 750 includes information identifying the time when the query was transmitted from the central unit to the medical monitoring device system. Query origin field 755 includes information identifying the cause of the query. For example, a query regarding the monitored individual's blood glucose level can be sent in response to the passage of a fixed period of time and a medication query may be sent in response to an anomalous measurement by the medical monitoring device system.

Nature of query field 760 includes information identifying the subject of the transmitted query. For example, nature of query field 760 can identify that the query is one or more of the queries in table 400 (FIG 4). Time response received field 765 can include information identifying the time when any response to the query was received. Response field 770 can include information identifying the response received. For example, response field 770 can identify that the monitored individual is not medicated or that the monitored individual has a certain blood pressure.

Libraries 705, 710 can be implemented in virtually any sort of data repository including databases, data tables, linked lists, or other associations of records 715, 720. Records 715, 720 can be data objects, data records, or other associations of information. Associations between data in libraries 705, 710 and records 715, 720 can be formed using, e.g., directories, files, filenames, or pointers. Libraries 705, 710 and records 715, 720 can be stored in a single data storage device or libraries 705, 710 and records 715, 720 can distributed among two or more data storage devices.

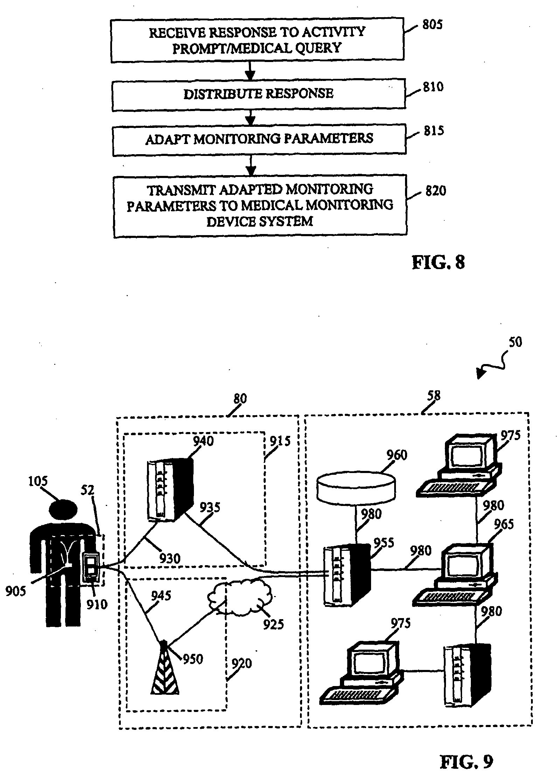

FIG. 8 shows a process 800 that can be performed by a central unit in response to receipt of a response to a medical query, receipt of a response to an activity prompt, or receipt of other information from a medical monitoring device system. For example, process 800 can be performed by central unit 58 (FIG. 2).

The system performing process 800 receives the response or other information at 805. The response can be received over a wired or a wireless data communications link.

In response to receipt of the response, the system can distribute the response to one or more third parties to whom the response is relevant at 810. For example, if the response indicates that a sample has been collected, the information regarding the collection can be distributed to a physician, a manager of a clinical trial, or other concerned party. In the case of sensor readings or other data samples, the distributed information can include the collected sample itself.

The system performing process 800 can also adapt the parameters of electrocardiographic monitoring performed by the medical monitoring device system at 815. The parameters can be adapted, e.g., to change thresholds at which disease states are identified, to change the algorithms used in monitoring, or to change the nature of the monitoring itself. The adaptation of the parameters is based on the response received from the medical monitoring device system to a query regarding a diabetic condition of the individual.

After adapting the parameters of the monitoring, the system can transmit the adapted parameters to the medical monitoring device system at 820. The transmission can result in the medical monitoring device system adapting the monitoring to accord with the transmitted parameters.

FIG. 9 shows another implementation of a medical monitoring system 50 for monitoring an individual 105. Medical monitoring system 50 includes medical monitoring device system 52 in communication with central unit 58 over one or more communication links 80.

Medical monitoring device system 52 can be adapted for electrocardiographic monitoring of an individual 105. Medical monitoring device system 52 can include a sensor module 905 and a monitor module 910. Sensor module 905 can include three ECG leads with electrodes, as well as a two channel ECG signal recorder and a wireless and/or wired data output. Sensor module 905 can also include a clip for attaching sensor module to a belt, a neckpiece, or other item worn by individual 105. Monitor module 910 includes a data input that is adapted to receive data output from sensor module 905 as well as one or more wireless and/or wired data outputs for data communication over communication link 80. Monitor module 910 also includes a data processing device that performs data processing activities in accordance with the logic of a set of machine-readable instructions. The instructions can be realized in digital electronic circuitry, integrated circuitry, specially designed ASICs (application specific integrated circuits), computer hardware, firmware, software, and/or combinations thereof. The instructions can describe how to identify and/or handle electrocardiogram data in accordance with one or more of the techniques described herein. Monitor module 910 can also include an input device and an output device for interaction with a user. The output device can present a prompt or a query to the monitored individual. The input device can receive information used in framing a response to a presented prompt or query.

Communication link 80 can include one or both of a wired data link 915 and a wireless data link 920 coupled to a data network 925 to place medical monitoring device system 52 in data communication with central unit 58. Wired data link 915 includes a public network portion 930 and a private or virtual private network portion
935 bridged by a server 940. Public network portion 930 provides for data communication between medical monitoring device system 52 and server 940 over a wired data link such as a telephone network. Private network portion 935 provides for private or virtually private data communication from server 940 to receiver 120. Server 940 can interface for data communication with both portions 930, 935. For example, server 940 can communicate directly with central unit 58 using the peer-to-peer protocol (PPP).

Wireless data link 945 can include one or more wireless receivers and transmitters 950 such as a WiFi receiver, a cellular phone relay station, and/or other cellular telephone infrastructure to place medical monitoring device system 52 in data communication with data network 925. In turn, data network 925 communicates with central unit 58.

Central unit 58 includes a receiver server 955, a data storage device 960, a call router 965, a communications server 970, and one or more application servers 975 that are all in data communication with one another over one or more data links 980. Receiver server 955 is a data processing device that receives and transmits communications over communications link 80 and relays incoming communications to data storage device 960 and call router 965 in accordance with the logic of a set of machine-readable instructions. Data storage device 960 is a device adaptable for the storage of information. For example, data storage device 960 can store one or more activity libraries 705 and/or medical libraries 710 (FIG. 7). Data storage device 960 can be a volatile and/or non-volatile memory that records information electrically, mechanically, magnetically, and/or optically. Call router 965 is a data processing device that, in accordance with the logic of a set of machine-readable instructions, identifies the content of an incoming communication and directs the communication to one or more appropriate application servers 975 based on that content. Communications server 970 is a data processing device that relays communications between call router 965 and one or more application servers 975 over an external network. Application servers 975 are data processing devices that interact with a user or operate in isolation to provide one or more monitoring services in accordance with the logic of a set of machine-readable instructions. Data links 980 can be part of a local area and/or private network or part of a wide area and/or public network.

In operation, sensor module 905 can sense, amplify, and record electrical signals relating to the activity of the heart. Sensor module 905 can also relay all or a portion of those signals to monitor module 910 where they can be managed. For example, monitor module 910 can manage the signals in accordance with one or more of processes 300 and 500 (FIGS. 3 and 5). As part of the management, monitor module 910 can transmit the signals to central unit 58. The signals can be transmitted alone or in association with other information. For example, the signals can be transmitted in association with other information that is responsive to queries or prompts.

The transmitted signals pass along communications link 80 over one or more of wired data link 915 and wireless data link 920 to central unit 58. At central unit 58, the signals are received by server 955 which causes at least a portion of the incoming signals to be stored on data storage device 960 and relayed to call router 965. The incoming signals stored on data storage device 960 can be stored any of a number of data structures

The incoming signals relayed to call router 965 can be distributed to one or more appropriate application servers 975. The distribution can be based on one or more different factors, including the load on certain application servers 975, the resources available at certain application servers 975 (e.g., the amount and/or type of memory available), or the content of the incoming signals. For example, when the signal relates to a certain category of cardiac event, the signal can be distributed to a certain application server 975 that is accessible to a cardiologist having expertise with that certain category of event. As another example, when the signal originates with an individual who is under the care of a particular physician, the signal can be distributed to a certain application server 975 that is accessible to that physician. As yet another example, when the signal relates to a certain category of cardiac event, the signal can be directed to a certain application server 975 that accesses an expert system or other set of instructions for diagnosing and/or treating that category of event. When appropriate, a signal can be routed to communications server 970 which in turn relays the signal to the appropriate application server 975 over an external network.

Communications can also be transmitted from central unit 58 back to individual 105 or to other individuals, as discussed above. As yet another example, when a physician or expert system identifies that care is needed, a message requesting that the individual seek care can be returned to individual 105 over communication link 80. In urgent care situations, third parties such as medical personnel can be directed to individual 105 by medical monitoring device system 52.

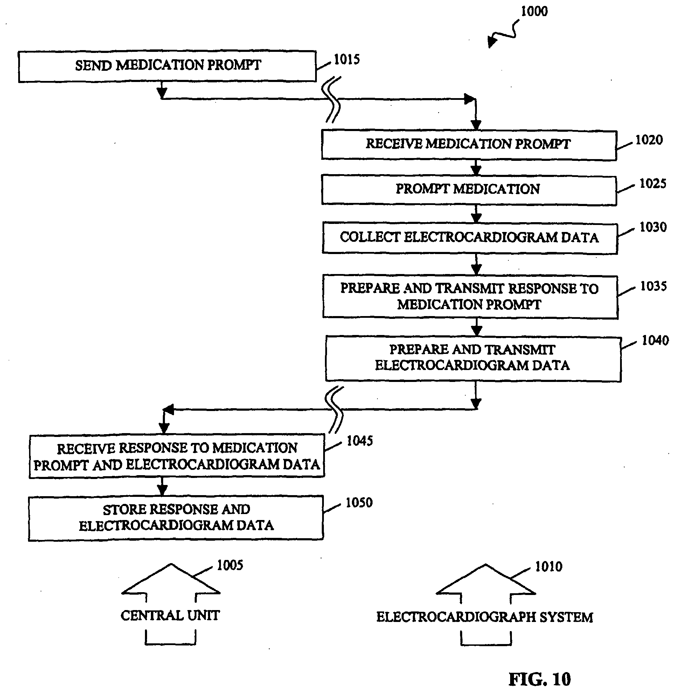

FIG 10 shows a process 1000 outside the scope of the present claims that can be performed using an electrocardiographic monitoring device during a clinical trial. A clinical trial is an experiment involving a set of human subjects having a clinical event as an outcome measure. Clinical trials are generally designed to yield information about the efficacy or safety of a drug, vaccine, diagnostic test, surgical procedure, or other medical intervention. For the sake of convenience, such interventions are referred to herein as "medication."

Aspects of process 1000 performed by a central unit such as central unit 58 are denoted by arrow 1005, whereas aspects of process 1000 performed by an electrocardiographic monitoring device are denoted by arrow 1010.

In process 1000, a central unit sends a medication prompt to an electrocardiograph system at 1015. The medication prompt is designed to trigger the monitored individual to medicate in accordance with the guidelines of the clinical trial. For example, the medication prompt can prompt the monitored individual to take a specified dosage of a specified pharmaceutical composition at a specified time. The medication prompt can be tailored to the monitored individual and based on factors such as the individual's weight, activity level, age, and medical history. The prompted medication need not be thought to have a direct consequence on cardiac function, but rather the prompted medication can be suspected of having a minimal effect or of having no effect on cardiac activity.

The transmitted medication prompt is received at the electrocardiogram system at 1020. The electrocardiogram system can then prompt the appropriate medication at 1025. For example, the electrocardiogram system can direct the monitored individual to dose with a specific medication in accordance with the clinical trial. The electrocardiogram system can collect electrocardiogram data before and after the dosage at 1030 as part of the clinical trial.

The medical monitoring device can also prepare and transmit a response to the medication prompt at 1035. The preparation of the response can be done automatically, i.e., without input from the individual who is monitored. Alternatively, the preparation of the response can be based on input from the individual who is monitored. For example, the medical monitoring device can present a text version of the medication prompt to the monitored individual and then receive a confirmation that the monitored individual has medicated as instructed.

The medical monitoring device can also prepare and transmit electrocardiogram data to the central unit at 1040. The transmitted data can be raw or processed data. The transmitted data can correspond to specific cardiac events of clinical significance or the transmitted data can correspond to a representative sample of the electrocardiogram data.

The central unit can receive the electrocardiogram data and the response to the medication prompt at 1045. The received data and response can be stored at 1050 and used to yield information about the efficacy or safety of the medication in the context of the clinical trial.

A number of implementations have been described. Nevertheless, it will be understood that various modifications may be made within the scope of the invention as defined by the following claims.

## Claims

1. A method comprising:
receiving a monitoring result from a remote monitoring device (52), the remote monitoring device (52) remotely monitoring a medical condition of an individual, wherein the remote monitoring device (52) is adapted for electrocardiographic monitoring of the individual;
receiving, from the remote monitoring device (52), a response to a query regarding a diabetic condition of the individual; and
adapting the monitoring of the medical condition of the individual by the remote monitoring device (52) with adapted parameters, the adapted parameters being based on the received response to the query regarding the diabetic condition of the individual.

2. The method of claim 1, wherein receiving the response comprises receiving a response to an individual-specific query tailored to the monitored individual.

3. The method of claim 1, further comprising storing the monitoring result and the response.

4. The method of claim 1, wherein adapting the monitoring comprises transmitting the adapted monitoring parameters to the remote monitoring device (52).

5. The method of claim 4, wherein the parameters are adapted to change thresholds at which a disease state is identified.

6. The method of claim 4, wherein the parameters are adapted to change the algorithms used in monitoring.

7. The method of claim 4, wherein transmitting the adapted monitoring parameters comprises transmitting adapted parameters for the identification of cardiac states to the remote monitoring device (52).

8. The method of claim 1, wherein:
the method further comprises distributing the response to one or more third parties to whom the response is relevant in response to receipt of the response.

9. A method performed by a medical monitoring device system, the method comprising:
monitoring a medical condition of an individual, the monitoring being initiated remotely by a monitoring service, wherein monitoring the medical condition comprises electrocardiographic monitoring of the individual;
receiving, from the monitoring service at the medical monitoring device system, a query relating to a diabetic condition of the individual;
transmitting a response to the query from the medical monitoring device system to the monitoring service;
receiving a prompt from the monitoring service, the prompt designed to provoke a predetermined action;
receiving, from the monitoring service, adapted parameters for the monitoring of the medical condition of the individual, the adapted parameters adapted based on the response to the query relating to the diabetic condition of the individual; and
adapting the monitoring of the medical condition in accordance with the received parameters.

10. The method of claim 9, further comprising transmitting a response to the prompt to the monitoring service.

11. The method of claim 9, wherein the received prompt is designed to provoke the monitored individual to undertake the predetermined action.

12. The method of claim 9, wherein receiving the prompt comprises receiving:
a medication prompt designed to provoke the monitored individual to medicate;
a sample prompt designed to provoke the collection of a sample;
an electrode check prompt designed to move the monitored individual to check an electrode;
a communications check prompt designed to move the monitored individual to ensure that communications with the monitoring service are maintained;
a battery check prompt designed to move the monitored individual to check a charge on the battery;
a physical activity prompt designed to move the monitored individual to curtail or halt physical activity; or
a prompt designed to move the monitored individual to lie down or sit down.

13. The method of claim 9, further comprising:
receiving input from the monitored individual; and
using the input in responding to the query.

14. An article comprising a machine-readable medium storing instructions operable to cause one or more devices to perform operations comprising:
monitoring a medical condition of an individual, wherein monitoring the medical condition comprises electrocardiographic monitoring of the individual;
receiving a query from a monitoring service, the query relating to a diabetic condition of the individual;
transmitting a response to the query to the monitoring service;
transmitting a result of the monitoring to the monitoring service;
receiving an activity prompt from the monitoring service, the activity prompt relating to an activity that is to be prompted;
prompting the activity related to the activity prompt;
transmitting a response to the prompt to the monitoring service;
receiving, from the monitoring service, adapted parameters for the monitoring of the medical condition of the individual, the adapted parameters being based on the response to the query relating to the diabetic condition of the individual; and
adapting the monitoring of the medical condition in accordance with the received parameters.

15. The article of claim 14, wherein prompting the activity comprises:
relaying the prompt to the monitored individual; and
receiving a response to the relayed prompt from the monitored individual.

16. The article of claim 14, wherein prompting the activity comprises provoking collection of a sample.

17. The article of claim 16, wherein provoking collection of the sample comprises provoking collection of the result of the monitoring.

18. The article of claim 14, wherein prompting the activity comprises provoking the monitored individual to medicate with a specific medication.

19. The article of claim 18, wherein provoking the monitored individual to medicate comprises provoking the monitored individual to medicate using a pharmaceutical composition having a specific color.

20. The article of claim 14, wherein transmitting the response comprises transmitting the
response without input from the monitored individual.

21. A device comprising:
an electrocardiograph configured to remotely monitor cardiac function of an individual;
a receiver configured to receive a communication from a remote monitoring service, wherein the receiver is configured to receive a query relating to a diabetic medical condition of the individual;
an output device configured to relay the communication to the monitored individual, wherein the output device is configured to display the query relating to the diabetic condition of the monitored individual;
an input device configured to receive an input responsive to the relayed communication from the monitored individual, wherein the input device is configured to receive an input relating to the diabetic condition; and
a transmitter configured to transmit a response to the communication to the remote monitoring service, the response based at least in part on the input received from the monitored individual;
wherein the receiver is configured to receive, from the remote monitoring service, adapted parameters for adapting the monitoring of the cardiac function of the individual, the adapted parameters being based on the response to the query relating to the diabetic condition of the individual.

22. The device of claim 21, wherein the receiver is to receive a prompt designed to move the monitored individual to a particular action.

## Patentansprüche

1. Verfahren, umfassend:
Empfangen eines Überwachungsergebnisses von einem Fernüberwachungsgerät (52), wobei das Fernüberwachungsgerät (52) eine Fernüberwachung eines Gesundheitszustandes eines Individuums durchführt, wobei das Fernüberwachungsgerät (52) zur elektrokardiografischen Überwachung des Individuums eingerichtet ist;
Empfangen einer Antwort von dem Fernüberwachungsgerät (52) auf eine Abfrage in Bezug auf einen diabetischen Zustand des Individuums; und
Anpassen der Überwachung des Gesundheitszustands des Individuums durch das Fernüberwachungsgerät (52) mit angepassten Parametern, wobei die angepassten Parameter auf der erhaltenen Antwort auf die Abfrage in Bezug auf den diabetischen Zustand des Individuums basieren.

2. Verfahren nach Anspruch 1, wobei Empfangen der Antwort das Empfangen einer Antwort auf eine individuumsspezifische Abfrage umfasst, die auf das überwachte Individuum zugeschnitten ist.

3. Verfahren nach Anspruch 1, weiterhin umfassend das Speichern des Überwachungsergebnisses und der Antwort.

4. Verfahren nach Anspruch 1, wobei das Anpassen der Überwachung das Übertragen der angepassten Überwachungsparameter an das Fernüberwachungsgerät (52) umfasst.

5. Verfahren nach Anspruch 4, wobei die Parameter angepasst sind, um Schwellenwerte zu ändern, bei denen ein Krankheitszustand identifiziert wird.

6. Verfahren nach Anspruch 4, wobei die Parameter angepasst sind, um die bei der Überwachung verwendeten Algorithmen zu ändern.

7. Verfahren nach Anspruch 4, wobei das Übertragen der angepassten Überwachungsparameter das Übertragen angepasster Parameter zum Identifizieren von Herzzuständen an das Fernüberwachungsgerät (52) umfasst.

8. Verfahren nach Anspruch 1, wobei:
das Verfahren weiterhin das Verteilen der Antwort an einen oder mehrere Dritte umfasst, für den/die die Antwort relevant ist.

9. Verfahren, ausgeführt von einem medizinischen Überwachungsgerätesystem, das Verfahren umfassend:
Überwachung eines Gesundheitszustands eines Individuums, wobei die Überwachung aus der Ferne durch einen Überwachungsdienst eingeleitet wird, wobei die Überwachung des Gesundheitszustands die elektrokardiografische Überwachung des Individuums umfasst;
Empfangen einer Abfrage in Bezug auf einen diabetischen Zustand des Individuums von dem Überwachungsdienst an dem medizinischen Überwachungsgerätesystem;
Übertragen einer Antwort auf die Abfrage von dem medizinischen Überwachungsgerätesystem an den Überwachungsdienst;
Empfangen einer Aufforderung von dem Überwachungsdienst, wobei die Aufforderung konzipiert ist, um eine vorbestimmte Aktion zu veranlassen;
Empfangen angepasster Parameter für die Überwachung des Gesundheitszustands des Individuums von dem Überwachungsdienst, wobei die angepassten Parameter auf der Antwort auf die Abfrage in Bezug auf den diabetischen Zustand des Individuums basieren; und
Anpassen der Überwachung des Gesundheitszustands in Übereinstimmung mit den empfangenen Parametern.

10. Verfahren nach Anspruch 9, weiterhin umfassend das Übertragen einer Antwort auf die Aufforderung an den Überwachungsdienst.

11. Verfahren nach Anspruch 9, wobei die empfangene Aufforderung konzipiert ist, um das überwachte Individuum zum Ausführen der vorbestimmten Aktion zu veranlassen.

12. Verfahren nach Anspruch 9, wobei das Empfangen der Aufforderung das Empfangen von Folgendem umfasst:
einer Medikamentenaufforderung, die konzipiert ist, um das überwachte Individuum zur medizinischen Behandlung zu veranlassen;
einer Probenaufforderung, die konzipiert ist, um die Abnahme einer Probe zu veranlassen;
einer Elektrodenprüfungsaufforderung, die konzipiert ist, um das überwachte Individuum zum Prüfen einer Elektrode zu veranlassen;
einer Kommunikationsprüfungsaufforderung, die konzipiert ist, um das überwachte Individuum zu veranlassen sicherzustellen, dass die Kommunikation mit dem Überwachungsdienst aufrechterhalten bleibt;
einer Batterieprüfungsaufforderung, die konzipiert ist, um das überwachte Individuum zum Prüfen einer Ladung der Batterie zu veranlassen;
einer körperlichen Aktivitätsaufforderung, die konzipiert ist, um das überwachte Individuum zum Einschränken oder Stoppen körperlicher Aktivität zu veranlassen; oder
einer Aufforderung, die konzipiert ist, um das überwachte Individuum zu veranlassen, sich hinzulegen oder hinzusetzen.

13. Verfahren nach Anspruch 9, weiterhin umfassend:
Empfangen einer Eingabe von dem überwachten Individuum; und
Verwendung der Eingabe als Antwort auf die Abfrage.

14. Gegenstand, umfassend ein maschinenlesbares Medium, das Anweisungen speichert, die funktionsfähig sind, um eine oder mehrere Vorrichtungen zu veranlassen, Vorgänge auszuführen, umfassend:
Überwachung eines Gesundheitszustands eines Individuums, wobei die Überwachung des Gesundheitszustands die elektrokardiografische Überwachung des Individuums umfasst;
Empfangen einer Abfrage von einem Überwachungsdienst, wobei sich die Abfrage auf einen diabetischen Zustand des Individuums bezieht;
Übertragen einer Antwort auf die Abfrage an den Überwachungsdienst;
Übertragen eines Ergebnisses der Überwachung an den Überwachungsdienst;
Empfangen einer Aktivitätsaufforderung von dem Überwachungsdienst, wobei die Aktivitätsaufforderung in Bezug zu einer Aktivität steht, für die eine Aufforderung erfolgen muss;
Aufforderung zu der Aktivität in Bezug auf die Aktivitätsaufforderung;
Übertragen einer Antwort auf die Aufforderung an den Überwachungsdienst;
Empfangen angepasster Parameter für die Überwachung des Gesundheitszustands des Individuums von dem Überwachungsdienst, wobei die angepassten Parameter auf der Antwort auf die Abfrage in Bezug auf den diabetischen Zustand des Individuums basieren; und
Anpassen der Überwachung des Gesundheitszustands in Übereinstimmung mit den empfangenen Parametern.

15. Gegenstand nach Anspruch 14, wobei die Aktivitätsaufforderung Folgendes umfasst:
Weiterleiten der Aufforderung an das überwachte Individuum; und
Empfangen einer Antwort auf die weitergeleitete Aufforderung von dem überwachten Individuum.

16. Gegenstand nach Anspruch 14, wobei die Aktivitätsaufforderung das Veranlassen der Abnahme einer Probe umfasst.

17. Gegenstand nach Anspruch 16, wobei das Veranlassen der Abnahme einer Probe das Veranlassen der Abnahme des Ergebnisses der Überwachung umfasst.

18. Gegenstand nach Anspruch 14, wobei die Aktivitätsaufforderung das Veranlassen des überwachten Individuums zur Behandlung mit einem bestimmten Medikament umfasst.

19. Gegenstand nach Anspruch 18, wobei das Veranlassen des überwachten Individuums zur Behandlung das Veranlassen des überwachten Individuums zur Behandlung mit einer pharmazeutischen Zusammensetzung, die eine spezifische Farbe aufweist, umfasst.

20. Gegenstand nach Anspruch 14, wobei das Übertragen der Antwort das Übertragen der Antwort ohne Eingabe von dem überwachten Individuum umfasst.

21. Vorrichtung, umfassend:
einen Elektrokardiographen, konfiguriert zur Fernüberwachung der Herzfunktion eines Individuums;
einen Empfänger, konfiguriert zum Empfangen einer Kommunikation von einem Fernüberwachungsdienst, wobei der Empfänger zum Empfangen einer Abfrage in Bezug auf einen diabetischen Gesundheitszustand des Individuums konfiguriert ist;
eine Ausgabevorrichtung, konfiguriert zum Weiterleiten der Kommunikation an das überwachte Individuum, wobei die Ausgabevorrichtung zum Anzeigen der Abfrage in Bezug auf den diabetischen Zustand des Individuums konfiguriert ist;
eine Eingabevorrichtung, konfiguriert zum Empfangen einer Eingabe als Antwort auf die weitergeleitete Kommunikation von dem überwachten Individuum, wobei die Eingabevorrichtung zum Empfangen einer Eingabe in Bezug auf den diabetischen Zustand konfiguriert ist; und
einen Sender, konfiguriert zum Übertragen einer Antwort auf die Kommunikation an den Fernüberwachungsdienst, wobei die Antwort mindestens teilweise auf der von dem überwachten Individuum empfangenen Eingabe basiert;
wobei der Empfänger zum Empfangen angepasster Parameter zum Anpassen der Überwachung der Herzfunktion des Individuums von dem Fernüberwachungsdienst konfiguriert ist, wobei die angepassten Parameter auf der Antwort auf die Abfrage in Bezug auf den diabetischen Zustand des Individuums basieren.

22. Verfahren nach Anspruch 21, wobei der Empfänger eine Aufforderung zu empfangen hat, die konzipiert ist, um das überwachte Individuum zum Ausführen einer bestimmten Aktion zu veranlassen.

## Revendications

1. Procédé comprenant :
la réception d'un résultat de surveillance provenant d'un dispositif de surveillance à distance (52), le dispositif de surveillance à distance (52) surveillant à distance un état médical d'un individu, dans lequel le dispositif de surveillance à distance (52) est conçu pour la surveillance d'électrocardiogramme de l'individu ;
la réception, depuis le dispositif de surveillance à distance (52), d'une réponse à une requête concernant un état diabétique de l'individu ; et
l'adaptation de la surveillance de l'état médical de l'individu par le dispositif de surveillance à distance (52) avec des paramètres adaptés, les paramètres adaptés étant basés sur la réponse reçue à la requête concernant l'état diabétique de l'individu.

2. Procédé selon la revendication 1, dans lequel la réception de la réponse comprend la réception d'une réponse à une requête spécifique à l'individu adaptée à l'individu surveillé.

3. Procédé selon la revendication 1, comprenant en outre le stockage du résultat de surveillance et la réponse.

4. Procédé selon la revendication 1, dans lequel l'adaptation de la surveillance comprend la transmission des paramètres de surveillance adaptés au dispositif de surveillance à distance (52).

5. Procédé selon la revendication 4, dans lequel les paramètres sont adaptés pour modifier des seuils auxquels un état pathologique est identifié.

6. Procédé selon la revendication 4, dans lequel les paramètres sont adaptés pour modifier les algorithmes utilisés dans la surveillance.

7. Procédé selon la revendication 4, dans lequel la transmission des paramètres de surveillance adaptés comprend la transmission des paramètres adaptés pour l'identification des états cardiaques au dispositif de surveillance à distance (52).

8. Procédé selon la revendication 1, dans lequel :
le procédé comprend en outre la diffusion de la réponse à une ou plusieurs parties tierces pour lesquelles la réponse est pertinente en réponse à la réception de la réponse.

9. Procédé exécuté par un système de dispositif de surveillance médicale, le procédé comprenant :
la surveillance d'un état médical d'un individu, la surveillance étant initiée à distance par un service de surveillance, dans lequel la surveillance de l'état médical comprend une surveillance d'électrocardiogramme de l'individu ;
la réception, depuis le service de surveillance au niveau du système de dispositif de surveillance médicale, d'une requête concernant un état diabétique de l'individu ;
la transmission d'une réponse à la requête provenant du système de dispositif de surveillance médicale au service de surveillance ;
la réception d'un message depuis le service de surveillance, le message étant conçu pour provoquer l'exécution d'une action prédéterminée ;
la réception, depuis le système de surveillance, de paramètres adaptés pour la surveillance de l'état médical de l'individu, les paramètres adaptés étant adaptés en fonction de la réponse à la requête concernant l'état diabétique de l'individu ; et
l'adaptation de la surveillance de l'état médical en fonction des paramètres reçus.

10. Procédé selon la revendication 9, comprenant en outre la transmission d'une réponse au message au service de surveillance.

11. Procédé selon la revendication 9, dans lequel le message reçu est conçu pour provoquer l'exécution par l'individu surveillé de l'action prédéterminée.

12. Procédé selon la revendication 9, dans lequel la réception du message comprend la réception :
d'un message pharmacologique conçu pour amener l'individu surveillé à prendre un médicament ;
d'un message de prélèvement conçu pour amener l'individu surveillé à effectuer un prélèvement d'échantillon ;
d'un message de contrôle d'électrode conçu pour que l'individu surveillé aille contrôler une électrode ;
d'un message de contrôle de communication conçu pour que l'individu surveillé aille vérifier que la communication avec le service de surveillance est maintenue ;
d'un message de contrôle de batterie conçu pour amener l'individu surveillé à contrôler une charge sur la batterie ;
d'un message d'activité physique conçu pour amener l'individu surveillé à réduire ou à arrêter une activité physique ; ou
d'un message conçu pour amener l'individu surveillé à s'allonger ou à s'asseoir.

13. Procédé selon la revendication 9, comprenant en outre :
la réception d'une saisie effectuée par l'individu surveillé ; et
l'utilisation de la saisie pour répondre à la requête.

14. Article comprenant un support lisible par machine stockant des instructions utilisables pour faire exécuter par un ou plusieurs dispositifs des opérations comprenant :
la surveillance d'un état médical d'un individu, dans lequel l'état médical comprend la surveillance d'électrocardiogramme de l'individu ;
la réception d'une requête provenant d'un service de surveillance, la requête concernant un état diabétique de l'individu ;
la transmission d'une réponse à la requête au service de surveillance ;
la transmission d'un résultat de la surveillance au service de surveillance ;
la réception d'un message d'activité provenant du service de surveillance, le message d'activité concernant une activité qui doit faire l'objet d'un guidage par message ;
le guidage par message de l'activité associée au message d'activité ;
la transmission d'une réponse au message au service de surveillance ;
la réception, provenant du service de surveillance, de paramètres adaptés pour la surveillance de l'état médical de l'individu, les paramètres adaptés étant basés sur la réponse à la requête concernant l'état diabétique de l'individu ; et
l'adaptation de la surveillance de l'état médical conformément aux paramètres reçus.

15. Article selon la revendication 14, dans lequel le guidage par message de l'activité comprend :
la transmission du message à l'individu surveillé ; et
la réception d'une réponse au message relayé provenant de l'individu surveillé.

16. Article selon la revendication 14, dans lequel le guidage par message de l'activité comprend la réalisation du prélèvement d'un échantillon.

17. Article selon la revendication 16, dans lequel la réalisation du prélèvement d'échantillon comprend la réalisation de la collecte du résultat de la surveillance.

18. Article selon la revendication 14, dans lequel le guidage par message de l'activité comprend la réalisation par l'individu surveillé de la prise d'un médicament spécifique.

19. Article selon la revendication 18, dans lequel la réalisation par l'individu surveillé de la prise d'un médicament comprend la réalisation par l'individu surveillé de la prise d'un médicament en utilisant une composition pharmaceutique ayant une couleur spécifique.

20. Article selon la revendication 14, dans lequel la transmission de la réponse comprend la transmission de la réponse sans saisie de la part de l'individu surveillé.

21. Dispositif comprenant :
un électrocardiographe configuré pour surveiller à distance la fonction cardiaque d'un individu ;
un récepteur configuré pour recevoir une communication provenant d'un service de surveillance à distance, dans lequel le récepteur est configuré pour recevoir une requête concernant une affection médicale diabétique de l'individu ;
un dispositif de sortie configuré pour relayer la communication à l'individu surveillé, dans lequel le dispositif de sortie est configuré pour afficher la requête concernant l'état diabétique de l'individu surveillé ;
un dispositif de saisie configuré pour recevoir une saisie en réponse à la communication relayée effectuée par l'individu surveillé, dans lequel le dispositif de saisie est configuré pour recevoir une saisie concernant l'état diabétique ; et
un émetteur configuré pour transmettre une réponse à la communication au service de surveillance à distance, la réponse étant basée au moins en partie sur la saisie reçue de l'individu surveillé ;
dans lequel le récepteur est configuré pour recevoir, depuis le service de surveillance à distance, des paramètres adaptés pour adapter la surveillance de la fonction cardiaque de l'individu, les paramètres adaptés étant basés sur la réponse à la requête concernant l'état diabétique de l'individu.

22. Dispositif selon la revendication 21, dans lequel le récepteur est destiné à recevoir un message conçu pour amener l'individu surveillé à effectuer une action particulière.
